Europäisches Patentamt

European Patent Office

Office européen des brevets

(11) Numéro de publication: **0 652 016 A1**

# (12) DEMANDE DE BREVET EUROPEEN

(21) Numéro de dépôt: **93420448.8**

(51) Int. Cl.6: **A61L 27/00**

(22) Date de dépôt: **10.11.93**

(43) Date de publication de la demande:
**10.05.95 Bulletin 95/19**

(84) Etats contractants désignés:
**AT BE CH DE DK ES FR GB GR IE IT LI LU MC NL PT SE**

(71) Demandeur: **PATRINOVE**
**39, rue du Lieutenant-Colonel-Prévost**
**F-69006 Lyon (FR)**
Demandeur: **TEXINFINE**
**60, rue Duguesclin**

**F-69006 Lyon (FR)**

(72) Inventeur: **Gutierrez, Gilles**
**39 Rue Lieutenant Colonel Prévost**
**F-69006 Lyon (FR)**

(74) Mandataire: **Monnier, Guy et al**
**Cabinet Lavoix Lyon**
**142-150 cours Lafayette**
**BP 3058**
**F-69392 Lyon Cédex 03 (FR)**

(54) **Obturateurs et bouchons de comblement centromédullaire et diaphysaire résorbables et radiostérillisables.**

(57) Ces bouchons de comblement centromédullaire et diaphysaire, résorbables et radiostérisables, sont essentiellement constitués d'acide désoxyribonucléique (ADN), éventuellement associé à un élément minéral ou organique choisi parmi le sodium, le potassium, le calcium, le magnésium, le fer, les amines.

Fig.1

EP 0 652 016 A1

Rank Xerox (UK) Business Services
(3. 10/3.09/3.3.4)

La présente invention concerne des obturateurs et bouchons de comblement centromédullaire et diaphysaire résorbables et radiostérilisables.

Lors de l'implantation d'une prothèse cimentée dans le canal médulaire d'un os, le chirurgien doit boucher ce canal afin d'empêcher le ciment de s'écouler et d'endommager la moelle.

Dans l'état actuel de la technique, les chirurgiens utilisent le plus souvent des bouchons constitués de collagène et d'hydroxyapatite. Ces substances présentent l'inconvénient de moduler l'expression phénotypique des ostéocytes qui se trouvent à leur contact. En effet, les radiographies des os fraîchement implantés d'un bouchon de collagène et d'hydroxyapatite ont permis de constater que l'opacité de la diaphyse osseuse est fortement diminuée à la hauteur du bouchon. Cette image traduit la perte de substances minérales.

La présente invention s'est donné pour objet d'apporter une solution à ce problème en proposant de nouveaux obturateurs et bouchons de comblement centromédullaire et diaphysaire résorbables et radiostérilisables.

L'inventeur a tout d'abord défini les critères auxquels devraient satisfaire les matériaux constitutifs d'un bouchon de comblement centromédullaire et diaphysaire ; il faut qu'ils soient :

- biodégradables
- radiostérilisables
- modelables en une structure apte à être introduite dans la cavité
- neutres vis-à-vis des cellules.

L'étude du mode d'action du collagène sur les cellules osseuses et sur les fibroblastes lui a permis de constater que ces cellules cherchaient toujours à renouveler la matrice extracellulaire dans laquelle elles se développaient.

En effet si l'on cultive des fibroblastes humains en présence de collagène bovin de type I (matériaux constitutifs des bouchons actuels), on constate une présence élevée de collagénases dans le milieu extracellulaire.

Or, l'os est composé, entre autres, d'une matrice extracellulaire de collagène I minéralisée par de l'hydroxyapatite, et ce collagène I est le collagène qui constitue la matrice extracellulaire des ostéoblastes.

L'inventeur a ensuite étudié l'activité stimulante de la synthèse des collagénases par le collagène minéralisé.

Les cellules étaient des fibroblastes provenant d'explants de peau humaine.

Les cellules sélectionnées ont été cultivées classiquement en milieu DMEM en présence de 5 % de sérum de veau foetal.

On a pris comme témoin des cellules cultivées en l'absence d'actif testé.

Les résultats donnant les valeurs obtenues après mise en culture de $0,5.10^6$ cellules sont rassemblés dans le tableau ci-après.

| | Nombre de cellules (1) à | | Collagénases à 48 h (2) | |
|---|---|---|---|---|
| | 24 heures | 48 heures | Activité (3) | ng / ml |
| Témoin | 0,490 ± 0,018 | 0,600 ± 0,015 | 420 ± 60 | 19 ± 2,5 |
| 10 $\mu$g/ml collagène + HO-AP (4) | 0,485 ± 0,014 | 0,490 ± 0,010 | 1 200 ± 80 | 95 ± 7 |

(1) : évalué par densité optique

(2) : méthode de dosage ELISA (immunofluorescence), modifiée comme suit :

Obtention des anticorps anticollagénases totaux : par immunisation d'un petit mamifère par des collagénases, gélatinase, stromelysine...

Purification : selon la méthode DOT-BLOT (fixation sur nitrocellulose) Révélation : anti-immunoglobuline spécifique fluorescente.

(3) : une unité de collagénase est définie comme la quantité d'enzymes nécessaire pour dégrader 1 $\mu$g de collagène natif par heure à 25 ° C.

(4) HO-AP = hydroxyapatite.

L'inventeur a étudié systématiquement, selon le même processus expérimental, de nombreuses substances naturelles, hémisynthétiques et synthétiques.

Chacune des substances présentant un intérêt a été radiostérilisée à 25 Kgray (2,5 mégarads), puis la matière, stérilisée par rayonnement gamma, a été à nouveau testée par le test décrit ci-avant, complété par un test M.T.T.

Ce test repose sur l'utilisation du sel de tétrazolium (jaune) qui sera réduit en un formazan (volet) par les réductases mitochondriales. Cette réduction est objectivée par une modification de la couleur du réactif qui passe du jaune au bleu.

Ce test permet d'évaluer globalement les capacités vitales des cellules. En effet, il est tout à fait possible que la diminution de collagénases soit due à une baisse d'activité et non pas à une modulation phénotypique.

Parmi les nombreuses substances ainsi examinées, l'inventeur a pu déterminer tout l'intérêt, pour la réalisation de bouchons de comblement centromédullaire et diaphysaire qui soient à la fois biodégradables, radiostérilisables, modelables en une structure apte à être introduite dans la cavité et neutres vis-à-vis des cellules, de l'acide désoxyribonucléique ou de ses dérivés.

Les obturateurs et bouchons de comblement centromédullaire et diaphysaire selon l'invention sont donc essentiellement constitués d'acide désoxyribonucléique ou de ses dérivés.

L'acide désoxyribonucléique (ADN) peut être obtenu sous différentes formes selon qu'il est salifié avec du sodium, un alcalin, un métal (salification partielle) ou complexé plus ou moins totalement avec un acide aminé.

La plupart de ces substances présentent un intérêt mais les difficultés d'obtention conduisent à un coût de production élevé qui ne se justifie pas toujours par une amélioration des performances.

Les complexes ADN-acides aminés tels que ADN-glycine,ADN-hydroxyproline, ADN-lysine et ADN-proline sont parmi les complexes les plus intéressants.

L'association d'un sel de l'ADN avec un sel dont la solution présente un pH acide est indispensable parce que les solutions aqueuses d'ADN de sodium développent un pH d'une alcalinité incompatible avec la vie cellulaire

Selon un mode de réalisation préféré de l'invention, l'acide désoxyribonucléique est utilisé sous forme de sel, tamponné à pH 6,5 ± 0,2.

Selon un mode de réalisation particulier de l'invention, le sel d'ADN est le désoxyribonucléate de sodium.

Il est tamponné à pH 6,5 ± 0,2 par de nombreuses substances, telles que le phosphate monosodique. On peut aussi citer également dans ce but et de manière non limitative tous les sels acides des polyoxyacides partiellement neutralisés, ainsi que les sels d'acides forts avec des bases faibles, de même que les combinaisons de ces sels.

Pour acidifier le milieu, on peut donc envisager d'utiliser les oxacides de métaux alcalins, alcalino-terreux ou autres qui soient susceptibles d'abaisser le pH d'une solution d'ADN, à l'exception des composés instables tels que $NaHCO$ qui alcalinisent le milieu après dégagement du $CO$ et les composés toxiques comme les sels d'acide borique. Les sels suivants conviennent tout particulièrement : $NaH_2PO_4$, $KH_2PO_4$, $NaHSO_4$, $KHSO_4$, $NH_3HSO_4$.

L'utilisation de phosphates calciques peut être envisagée, en tant qu'éléments précurseurs de l'hydroxyapatite. Toutefois, l'emploi du $CaHPO_4$ est délicat en raison de sa solubilité. Les polyacides carboxyliques, tels que l'acide citrique, donnent d'assez bons résultats sous la forme de citrate monosalifié ou partiellement neutralisé par un cation bivalent.

La présente invention sera mieux comprise et ses avantages ressortiront bien de l'exemple qui suit, accompagné du dessin annexé dans lequel :

Figure 1 représente une vue en perspective d'un mode de réalisation du bouchon selon l'invention et

Figure 2 représente, en coupe, l'implantation du bouchon dans le canal médullaire d'un fémur.

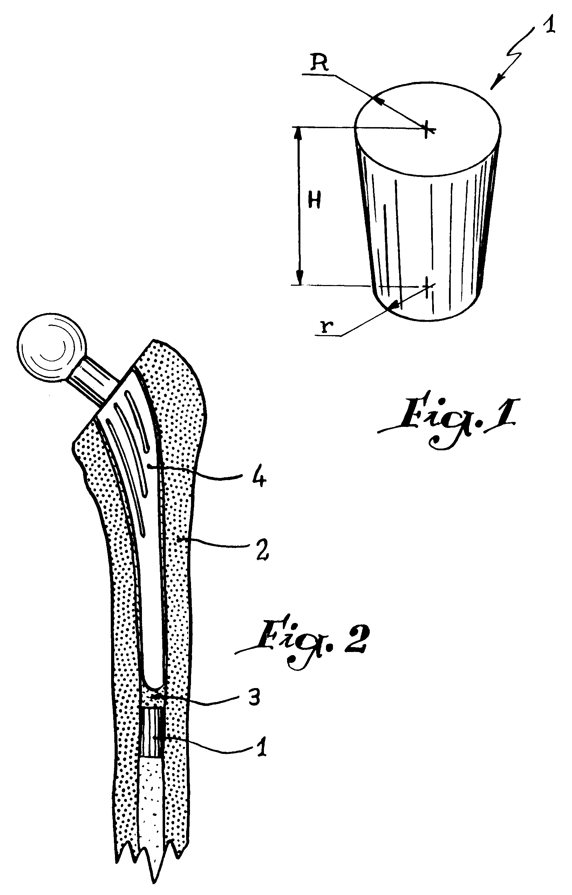

Sur la figure, 1 désigne le bouchon diaphysaire selon l'invention, l'os étant désigné par 2, le canal médulaire par 3, et la prothése par 4.

On réalise une poudre par broyage de 96,64 % de fibres d'ADN sodique dans de l'azote liquide en présence de 3,26 % de $NaH_2PO_4$. Le pH de 1 g du mélange dans 100 ml d'eau est de 6,5. On comprime cette poudre pour obtenir le bouchon 1 représenté au dessin.

Il s'agit, dans le mode de réalisation représenté, d'un cône tronqué dont le volume est défini par la formule suivante :

$$V = \pi . (H/3) . (R^2 = r^2 + Rr)$$

dans laquelle H est la hauteur du bouchon,

R le grand rayon et

r le petit rayon (ici la base).

La surface décrite par R peut être munie d'un dispositif (non représenté au dessin) permettant une meilleure préhension.

Il est bien évident que, sans sortir du domaine de l'invention, les volumes les plus divers peuvent être envisagés et l'on peut citer, à titre d'exemple non limitatif, quelques volumes "classiques" tels que les sphères, les rhomboèdres, les parallélépipèdes, les tétraèdres, les prismes, les pyramides entières ou tronquées, les tonneaux.

Des volumes associant un cylindre ou un cône tronqué semblent les mieux adaptés, mais on peut aussi utiliser des formes plus diversifiées.

Le créateur d'une forme de bouchon centromédullaire ou diaphysaire devra tenir compte de deux phénomènes.

On a tout d'abord une expansion du bouchon après son formage par compression, puis une rétraction de la matière après radiostérilisation.

Dans le cas de la formule précitée , on tient compte d'une expansion de 5,5 % et d'une rétraction de 4 à 8 % après radiostérilisation. Ces coefficients ne sont bien entendu donnés qu'à titre indicatif ils dépendent en effet de la force de compression et du débit-dose de la source de rayonnement.

Dans l'exemple donné, une force équivalente à l'application d'un poids de 250 Kg par cm2 a été utilisée pour obtenir des bouchons de bonne tenue.

Une adaptation de cette technique permet d'utiliser des forces de compression beaucoup plus faibles. Elle consiste à humecter le bouchon après qu'il ait été formé. Il se forme alors à la surface un revêtement vitreux très dur après séchage qui constitue une enveloppe rigide et lisse du bouchon.

La biodégradabilité du matériau a été testée par des essais chez l'animal. L'implantation de bouchons de 1 gramme dans le péritoine de souris n'a enclanché aucune réaction particulière sur des animaux d'un poids moyen de 50 grammes (n = 8).

On a pu noter que la matière se dégradait très rapidement lorsqu'elle servait de support à la prolifération cellulaire dans un milieu de culture (T = 6 heures) et que les bouchons de 1 gramme disparaissaient avec un T 1/2 = 6 heures lorsqu'ils étaient implantés chez l'animal.

Chez l'homme, l'utilisation de ces obturateurs pour contenir les ciments acryliques des prothèses de hanche implantées dans le fût fémoral afin que le ciment n'occupe pas la totalité de la cavité médulaire a fait l'objet de nombreuses observations au cours desquelles on a pu constaté l'excellente tolérance, la bonne biodégradabilité, l'absence de corticalisation du fût fémoral et l'absence d'ostéoporose sur la corticale.

L'utilisation de bouchons ou d'obturateurs de taille inférieure pour obturer des cavités dans le maxillaire, l'humérus et le tibia a fait l'objet d'observations identiques.

**Revendications**

1.  Obturateurs et bouchons de comblement centromédullaire et diaphysaire, caractérisés en ce qu'ils sont essentiellement constitués d'acide désoxyribonucléique (ADN)

2.  Obturateurs et bouchons selon la revendication 1, caractérisés en ce que l'acide désoxyribonucléique est associé à un élément minéral ou organique choisi parmi le sodium, le potassium, le calcium, le magnésium, le fer, les amines.

3.  Obturateurs et bouchons selon la revendication 1 et la revendication 2, caractérisés en ce que l'acide désoxyribonucléique a un pH stabilisé entre 6 et 7,5 par association d'un sel, d'un polyacide partiellement neutralisé, ou d'un acide fort et d'une base faible.

4.  Obturateurs et bouchons selon la revendication 3, caractérisés en ce que l'acide désoxyribonucléique est utilisé sous forme de sel, tamponné à pH 6,5 ± 0,2.

5.  Obturateurs et bouchons selon la revendication 4, caractérisés en ce que le sel d'ADN est le désoxyribonucléate de sodium.

6.  Obturateurs et bouchons selon la revendication 2, caractérisés en ce qu'ils sont constitués d'un complexe choisi parmi les complexes ADN-acides aminés tels que ADN-glycine,ADN-hydroxyproline, ADN-lysine et ADN-proline.

7.  Obturateurs et bouchons selon l'une quelconque des revendications 1 à 6, caractérisés en ce qu'ils sont humectés de façon qu'il se forme à leur surface, après séchage, un revêtement vitreux très dur constituant une enveloppe rigide et lisse.

R

H

r

1

Fig.1

4

2

Fig.2

3

1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | FR-A-2 570 606 (LABORATOIRE LANDANGER) <br> * le document en entier * <br> ----- | 1 | A61L27/00 |

**DOMAINES TECHNIQUES RECHERCHES (Int.Cl.6)**

A61L

Le présent rapport a été établi pour toutes les revendications

| Lieu de la recherche | Date d'achèvement de la recherche | Examinateur |
|---|---|---|
| LA HAYE | 11 Avril 1994 | Peltre, C |

EPO FORM 1503 03.82 (P04C02)